(19) Europäisches Patentamt
European Patent Office
Office européen des brevets

(11) **EP 2 956 188 B1**

(12) **EUROPÄISCHE PATENTSCHRIFT**

(45) Veröffentlichungstag und Bekanntmachung des Hinweises auf die Patenterteilung:
**05.07.2017 Patentblatt 2017/27**

(21) Anmeldenummer: **14703320.3**

(22) Anmeldetag: **04.02.2014**

(51) Int Cl.:
***A61M 1/16*** *(2006.01)*

(86) Internationale Anmeldenummer:
**PCT/EP2014/052087**

(87) Internationale Veröffentlichungsnummer:
**WO 2014/124832 (21.08.2014 Gazette 2014/34)**

(54) **VORRICHTUNG ZUR REGELUNG EINER BEHANDLUNGSVORRICHTUNG**

DEVICE FOR CONTROLLING A HANDLING DEVICE

DISPOSITIF DE RÉGULATION D'UN DISPOSITIF DE TRAITEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.02.2013 DE 102013002395**
**13.02.2013 US 201361764223 P**

(43) Veröffentlichungstag der Anmeldung:
**23.12.2015 Patentblatt 2015/52**

(73) Patentinhaber: **Fresenius Medical Care Deutschland GmbH**
**61352 Bad Homburg (DE)**

(72) Erfinder:
• **HEIDE, Alexander**
**65817 Eppstein (DE)**
• **NIKOLIC, Dejan**
**65824 Schwalbach am Taunus (DE)**
• **PETERS, Arne**
**61352 Bad Homburg (DE)**
• **WIKTOR, Christoph**
**63571 Gelnhausen (DE)**

(74) Vertreter: **Ziermann, Oliver**
**Fresenius Medical Care AG & Co. KGaA**
**Global Patents & IP**
**Else-Kröner-Strasse 1**
**61352 Bad Homburg (DE)**

(56) Entgegenhaltungen:
**EP-A1- 0 516 152      WO-A1-00/06217**
**WO-A1-2004/004804      WO-A1-2013/164089**
**DE-A1- 3 020 756      DE-A1- 3 600 227**
**DE-C1- 19 823 811**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

## Technisches Gebiet

Die vorliegende Erfindung betrifft eine Vorrichtung zur Regelung einer Behandlungsvorrichtung, insbesondere zur Regelung der Ultrafiltration bei einer Dialysebehandlung.

## Hintergrund

Die Dialyse ist ein Verfahren zur Blutreinigung von Patienten mit akuter oder chronischer Niereninsuffizienz. Grundsätzlich unterscheidet man hierbei zwischen Verfahren mit einem extrakorporalen Blutkreislauf, wie der Hämodialyse, der Hämofiltration oder der Hämodiafiltration und der Peritonealdialyse, die keinen extrakorporalen Blutkreislauf aufweist.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, tritt Wasser aus dem Patientenblut über die Dialysemembran in den Dialysatkreislauf über, das sogenannte Ultrafiltrat. Dieser Vorgang der Ultrafiltration führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators zugesetzt wird oder stromabwärts, spricht man von Prä- oder Postdilution. Der Stoffaustausch erfolgt bei der Hämofiltration konvektiv.

Eine Kombination der Hämodialyse und der Hämofiltration liegt vor, wenn bei einer Dialysebehandlung dem Patientenblut gleichzeitig Substituat zugeführt wird. Diese Behandlungsform, die auch als Hämodiafiltration bezeichnet wird soll im Folgenden von dem Begriff der Hämodialyse, der Dialyse oder der Dialysebehandlung umfasst sein.

Bei der Dialysebehandlung ist es von entscheidender Bedeutung, dass der Flüssigkeitsentzug mit großer Genauigkeit gemessen und bilanziert wird, denn schon ein geringfügig zu großer Flüssigkeitsentzug könnte gravierende Folgen für den Patienten haben.

Dies wird dadurch sichergestellt, dass der Zufluss des Dialysats oder der Dialysierflüssigkeit in die Dialysierflüssigkeitskammer und der Abfluss der Dialysierflüssigkeit aus der Dialysierflüssigkeitskammer getrennt von einander kontrolliert werden. Die Bilanz zwischen der der Dialysierflüsigkeitskammer zugeführten Flüssigkeitsmenge und der aus der Dialysierflüssigkeitskammer abgeführten Flüssigkeitsmenge gibt dabei gleichzeitig ein Maß für die Menge des dem Patientenblut entzogenen Ultrafiltrats an.

Eine Möglichkeit zur Bilanzierung ist der Einsatz von Bilanzkammern, die auf dem Prinzip beruhen, dass eine zugeführte Flüssigkeitsmenge in einem Zufluss zur Dialyiserflüssigkeitskammer einer in einen Abfluss aus der Dialysierflüssigkeitskammer abgeführten Flüssigkeitsmenge entspricht.

Für das zusätzliche Entziehen von Flüssigkeit aus dem Patienten wird ein weiterer Flussweg mit Fördereinrichtung, die sog. Ultrafiltrationspumpe, parallel zur Bilanzkammer angeordnet. Die abzuziehende Flüssigkeit wird dabei über den parallelen Flussweg an der Bilanzkammer vorbei und durch die Ultrafiltrationspumpe gemessen und bildet so ein Maß für die Flüssigkeitsbilanz.

Bilanzkammern sind konstruktiv aufwendig und stellen hohe Anforderungen an die Fertigungstoleranz.

Alternativ kann die Kontrolle der Ultrafiltration durch die Kontrolle der Flussrate in der Zuleitung zur Dialysierflüssigkeitskammer und der Flussrate in der Ableitung aus der Dialysierflüssigkeitskammer durch unabhängig voneinander ansteuerbare jeweils in der Zuleitung und in der Ableitung angeordnete Pumpen erfolgen. In diesem Fall erfolgt die Bilanzierung durch jeweilige in der Zu- und in der Ableitung angeordnete Flusssensoren oder Waagen, was mit einem hohen Aufwand für die Kalibrierung dieser Sensoren oder Waagen verbunden ist.

WO 00/06172 A1 offenbart eine Vorrichtung mit einem hydraulischen Ultrafiltrationskreislauf, der eine erste und eine zweite Verdrängerpumpe, die Dialysat zu oder von einer Dialysierflüssigkeitskammer eines Dialysators pumpen. Zwischen der jeweiligen Pumpe und dem entsprechenden Zugang des Dialysators ist ein entsprechender Flussmesser angeordnet, der die Flussrate der entsprechenden Pumpe bestimmt. Der Dialysator ist ebenfalls an einen Kreislauf angeschlossen, der eine physiologische Flüssigkeit (z.B. Blut) extrakorporal von einer Quelle (z.B. einem Patienten) durch einem Blutabteil eines Dialysators und zurück zu der Quelle pumpt. In Abhängigkeit von den relativen Pumpraten der ersten und der zweiten Pumpe wird eine Ultrafiltrationsbedingung erreicht, die einen Nettoflüssigkeitsfluss entweder von dem Dialysat in die physiologische Flüssigkeit oder von der physiologischen Flüssigkeit in das Dialysat ergibt. Die Flussmesser ermöglichen ein Bestimmung des Flüssigkeitsflusses und erlauben eine Ultrafiltration mit einer hohen Genauigkeit, die ohne eine zusätzliche Ultrafiltrationspumpe in dem Ultrafiltrationskreislauf auskommt.

In der DE 3600227 A1 wird eine Ultrafiltrationsregeleinrichtung offenbart, bei der die in den Dialysator eintretende Flüssigkeitsmenge über einen Flussmesser messbar und über eine vor dem Dialysator angeordnete Pumpe konstant regelbar und die aus dem Dialysator austretende Flüssigkeitsmenge über eine zweiten Flussmesser messbar und über einen hinter dem Dialysator angeordnete zweite Pumpe derart regelbar ist, dass die Differenz der Flüssigkeitsmengen dem über die semipermeable Membran diffundierenden gewünschten Ultrafiltrationsvolumen entspricht.

Die in der EP 0516 152 A1 offenbarte Vorrichtung enthält einen Dialysator, der einen extrakorporalen Blutkreislauf von einem Kreislauf trennt, in dem Dialysierflüssigkeit zirkuliert, einen Differenzflussmesser mit zwei Röhren, die in einem Gegenstrom durch eine Flüssigkeit durchströmt werden, die in einem ersten Kanal Flüssigkeit zu dem Dialysator und in einem zweiten Kanal Flüssigkeit von dem Dialysator transportiert, eine elektrischen Einheit, die ein Signal proportional zur Massenstromdifferenz zwischen diesen Flüssen erzeugt, eine dritte Röhre, die von der ersten Röhre abzweigt, die eine Flüssigkeitsmenge in das Blut infundiert und eine zentrale Kontrolleinheit, die das von der Einheit erzeugte Signal verarbeitet und die eine in der zweiten Leitung angeordnete Pumpe derart kontrolliert, dass durch diese hindurch eine Flüssigkeitsmenge fließt, die eine Summe ist aus: dem Flüssigkeitsflusses zu dem Dialysator, der Flüssigkeitsmenge, die dem Patienten entzogen wird und die dem vorbestimmten Gewichtsverlust entspricht, sowie einer Wassermenge, die gleich der Menge der infundierten Flüssigkeit ist.

Es ist daher eine Aufgabe der Erfindung, zumindest eine der oben genannten Schwierigkeiten zu überwinden und eine einfache Vorrichtung und ein entsprechendes Verfahren zur Regelung der Ultrafiltration zur Verfügung zu stellen.

## Zusammenfassung

Diese Aufgabe wird gelöst durch eine Vorrichtung zur Regelung einer Ultrafiltration bei einer Dialysebehandlung bei der zu ultrafiltrierendes Blut in einem extrakorporalen Blutkreislauf eine Blutkammer eines durch eine semipermeable Membran in die Blutkammer und eine Dialysierflüssigkeitskammer unterteilten Dialysators durchströmt und Dialysierflüssigkeit in einem Dialysierflüssigkeitskreislauf die Dialysierflüssigkeitskammer des Dialysators durchströmt. Die offenbarungsgemäße Vorrichtung weist eine Blutpumpe zur Erzeugung eines Blutflusses in dem extrakorporalen Blutkreislauf, eine Dialysierflüssigkeitstpumpe zur Erzeugung eines Dialysierflüssigkeitsflusses in dem Dialysierflüssigkeitskreislauf, eine Bilanziervorrichtung zum Aufstellen einer Flüssigkeitsbilanz im Dialysierflüssigkeitskreislauf zwischen einem Zustrom und einem Abfluss aus der Dialysierflüssigkeitskammer als Maß für die Ultrafiltration, sowie einer Regeleinheit zum Regeln der Blutpumpe und/oder der Diaylsierflüssigkeitspumpe auf. Die Regelung der Blutpumpe und/oder der Dialysierflüssigkeitspumpe erfolgt so, dass eine vorbestimmte Ultrafiltration ohne weitere aktive Steuerung oder Regelung des aus dem Dialysator abfließenden Dialysierflüssigkeitsflusses erzielt wird.

Die Dialysierflüssigkeitspumpe ist im Dialysierflüssigkeitskreislauf in einer Zuleitung zu der Dialysierflüssigkeitskammer angeordnet. Auf diese Weise kann die Dialysierflüssigkeitspumpe nahe an einer Diaylsierflüssigkeitszubereitung angeordnet werden.

Weiterhin wird die vorliegende Aufgabe durch eine Vorrichtung nach Anspruch 1 gelöst. Vorteilhafte Ausführungsformen sind in den abhängigen Ansprüchen angegeben.

Die Erfinder haben erkannt, dass bei dieser Konfiguration keine weitere aktive Steuerung oder Regelung des Dialysierflüssigkeitsflusses stromab der Dialysierflüssigkeitskammer erforderlich ist. Dies geht mit einem verminderten konstruktiven Aufwand einher.

Die Regelung der Ultrafiltration, insbesondere der Ultrafiltrationsrate oder des Ultrafiltrationsvolumens kann in diesem Fall ausschließlich durch die Steuerung und/oder Regelung der Blutpumpe und der Dialsierflüssigkeitspumpe erfolgen.

Weiterhin vorteilhaft ist die Blutpumpe im Blutkreislauf in einer Zuleitung zu der Blutkammer angeordnet. Auf diese Weise trägt der durch die Blutpumpe bereitgestellte Druck zu einem Überdruck in der Blutkammer gegenüber der Dialysierflüssigkeitskammer bei.

Die Regelung der Ultrafiltration kann so erfolgen, dass für die Blutflussrate oder für den von der Blutpumpe aufgebrachten Druck ein vorbestimmter Wert einstellbar ist und die Regeleinheit die Ultrafiltration dadurch regelt, dass die Dialysierflüssigkeitspumpe in Abhängigkeit von einer gemessenen Ultrafiltrationsrate geregelt wird, etwa indem ein Druck oder ein Volumenstrom in einer Zuleitung zu der Dialysierflüssigkeitskammer im Dialysierflüssigkeitskreislauf entsprechend geregelt wird.

Alternativ kann die Regelung der Ultrafiltration auch so erfolgen, dass für die Dialysierflüssigkeitsrate oder für den Förderdruck der Dialysierflüssigkeitspumpe ein vorbestimmter Wert einstellbar ist und die Regeleinheit die Ultrafiltration dadurch regelt, dass sie die Blutpumpe in Abhängigkeit von einer gemessenen Ultrafiltrationsrate regelt, indem der Druck oder der Volumenstrom in einer Zuleitung zu der Blutkammer im extrakorporalen Blutkreislauf entsprechend geregelt wird.

Es ist aber jede andere Regelung der Ultrafiltration möglich, so lange durch die Blutpumpe im Blutkreislauf und durch die Dialysierflüssigkeitspumpe im Dialysierflüssigkeitskreislauf die Druckverhältnisse am Dialysator so gesteuert und/oder geregelt werden können, dass eine gewünschte Ultrafiltration stattfindet.

In einer Weiterbildung der Vorrichtung weist die Bilanziervorrichtung eine Differenzflussmesseinheit zum Messen des Differenzflusses zwischen einem Fluss in dem Zustrom zur Dialysierflüssigkeitskammer und dem Abfluss aus der Dialysierflüssigkeitskammer, eine Verzweigung von dem Zufluss oder dem Abfluss zur Abzweigung von Dialysierflüssigkeit von dem Zufluss oder dem Abfluss in einen weiteren Flussweg, sowie eine Einrichtung zur Einstellung der Flussmenge in dem Zufluss, in dem Abfluss und/ oder in dem weiteren Flussweg auf, die derart ansteuerbar ist, dass der gemessene Differenzfluss eine vorbestimmte Bedingung erfüllt. In dieser Weiterbildung weist die Vorrichtung weiterhin eine Einrichtung zur Ermittlung der Flussmenge in dem weiteren Flussweg als Maß der Flüssigkeitsbilanz auf.

Die Flussmenge in dem Zufluss oder dem Abfluss aus der Dialysierflüssigkeitskammer kann etwa mit Hilfe einer im Zufluss oder im Abfluss angeordneten Dialysierflüssigkeitspumpe eingestellt werden. Der Fluss in dem weiteren Flussweg kann mit einer in dem Flussweg angeordneten Pumpe eingestellt werden.

## Kurzbeschreibung der Figuren

Fig. 1 zeigt ein Blockdiagramm eines Dialysegeräts mit einer Vorrichtung zur Regelung der Ultrafiltration.

Fig. 2 zeigt ein Blockdiagram eines weiteren Dialysegeräts mit einer weiteren Vorrichtung zur Regelung der Ultrafiltration

Fig. 3 zeigt ein Blockdiagramm eines Ersatzschaltbildes für eine Dialysevorrichtung

Fig. 4 zeigt ein Blockdiagramm eines vereinfachten elektrischen Ersatzschaltbildes für eine Dialysevorrichtung.

## Detaillierte Beschreibung der Figuren

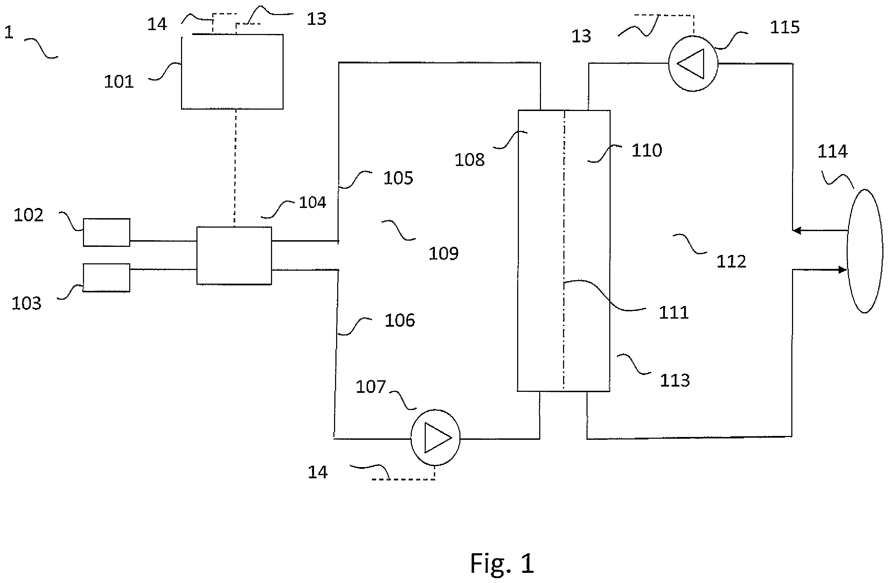

In Figur 1 ist schematisch ein Dialysegerät 1 mit einer Vorrichtung zur Regelung der Ultrafiltration im Einklang mit der Lehre der vorliegenden Erfindung dargestellt. Das zu behandelnde Blut wird über einen Zugang 114 dem Patienten entnommen und mit einer Blutpumpe 115 im extrakorporalen Blutkreislauf 112 durch eine Blutkammer des Dialysators 113 und über den Zugang 114 an den Patienten zurückgegeben. Der Zugang 114 verbindet den Blutkreislauf 112 mit einem Blutgefäß des Patienten, das zur Blutentnahme und Rückgabe geeignet ist. Der Zugang 114 kann zur Blutentnahme und zur Rückgabe des Blutes einen getrennten Ab- und Zufluss enthalten ('Double Needle'- Verfahren), oder Zu- und Abfluss können als ein Element ausgeführt sein ('Single Needle'- Verfahren).

Im Dialysator 113 trennt eine semipermeable Membran 111 eine Dialysierflüssigkeitskammer 108 von einer Blutkammer 110. Über die semipermeable Membran 111 findet ein Flüssigkeits- und Stoffaustausch von der Blutkammer 110 in die Dialysierflüssigkeitskammer 108 statt. Durch die Dialysierflüssigkeitskammer 108 des Filters 113 wird Dialysierflüssigkeit im Dialysierflüssigkeitskreislauf 109 mit einer Dialysierflüssigkeitspumpe 107 in einer Zuleitung 106 stromauf der Dialysierflüssigkeitskammer transportiert. Alternativ kann die Dialysierflüssigkeitspumpe auch in einer Ableitung 105 stromab der Dialysierflüssigkeitskammer angeordnet sein. Im von einer Dialysierflüssigkeitsquelle 103 gespeisten Dialysierflüssigkeitskreislauf 109 ist eine Bilanziervorrichtung 104 angeordnet, zur Bilanzierung der der Dialysierflüssigkeitskammer zugeführten und der aus dem Dialysator abfließenden Dialysierflüssigkeit. Hierzu kann die Flussrate im Zufluss zur Dialysierflüssigkeitskammer und die Flussrate im Abfluss der Dialysierflüssigkeitskammer separat erfasst werden, oder es kann ein Differenzfluss als Maß für die Flüssigkeitsbilanz ermittelt werden. Die Flüssigkeitsbilanz entspricht der über die Membran im Dialystor entzogenen Ultrafiltrationsmenge. Aus dem Dialysator abfließende, sogenannte verbrauchte Dialysierflüssigkeit wird im Allgemeinen in einen Dialysierflüssigkeitsabfluss 102 verworfen. Alternativ kann eine Regeneration verbrauchter Dialysierflüssigkeit vorgesehen sein.

Durch die Steuerung der Blutpumpe 115 und die Steuerung der Dialysierflüssigkeitspumpe 107 werden die Druckverhältnisse an der Membran 111 im Dialysator 113 so beeinflusst, dass in der Blutkammer 110 gegenüber der Dialysierflüssigkeitskammer 108 ein Überdruck herrscht. Dadurch erfolgt ein Transport von Flüssigkeit durch die Membran von der Blutkammer 110 in die Dialysierflüssigkeitskammer 108.

Die Blutpumpe kann zum Erzielen einer bestimmten Pumpendrehzahl oder einer bestimmten Blutflussrate als Betriebsparameter ansteuerbar sein, etwa in einer Ausführung als peristaltische Pumpe. Alternativ kann die Blutpumpe zum Erzielen eines bestimmten Förderdrucks als Betriebsparameter ansteuerbar sein, etwa als Impellerpumpe.

Ebenso kann die Dialysierflüssigkeitspumpe zum Erzielen einer bestimmten Förderrate oder Pumpendrehzahl als peristaltische Pumpe, als Membranpumpe, als Kolbenpumpe o.ä. ausgeführt sein oder zum Aufbauen eines bestimmten Förderdrucks, z.B. als Impellerpumpe.

Eine mit der Bilanziervorrichtung 104 über eine Messleitung verbundene Steuer- und Regeleinheit 101 ist über die Steuerleitung 13 mit der Blutpumpe 115 und über die Steuerleitung 14 mit der Dialysierflüssigkeitspumpe 107 verbunden. Während der Blutbehandlung werden kontinuierlich oder periodisch aktuelle Messparameter der Ultrafiltration, etwa der Ultrafiltrationsmenge oder Ultrafiltrationsrate von der Bilanziervorrichtung an die Steuer- und Regeleinheit 101 übermittelt. Die Steuer- und Regeleinheit 101 verwendet die aktuellen Messparameter um Steuersignale für die die Blutpumpe 115 sowie für die Dialysierflüssigkeitspumpe 107 abzuleiten. Die Ansteuerung der Dialysierflüssigkeitspumpe 107 und der Blutpumpe 115 erfolgt dabei im Hinblick auf eine zu erzielende Ultrafiltration, etwa eine bestimmte Ultrafiltrationsrate oder ein bestimmtes, über den Behandlungsverlauf zu erzielendes Ultrafiltrationsvolumen.

Die Regelung kann etwa so erfolgen, dass die Blutpumpe 115 mit einer konstanten Drehzahl oder mit einem konstanten Förderdruck betrieben wird, und die Dialysierflüssigkeitspumpe 107 so gesteuert wird, dass der von der Bilanziervorrichtung 104 übermittelte Ultrafiltrationswert als Regelgröße dient. Liegt etwa der übermittelte Wert der Ultrafiltrationsrate über einem entsprechenden Sollwert, so ist die Dialysierflüssigkeitspumpe 107 zu beschleunigen, liegt die Ultrafiltrationsrate unter ihrem Sollwert, wird die Dialysierflüssigkeitspumpe 107 gedrosselt.

Eine alternative Regelstrategie kann sein, die Dialysierflüssigkeitspumpe 107 mit einer konstanten Drehzahl oder mit einem konstanten Förderdruck zu betreiben, und die Blutpumpe 115 so anzusteuern, dass der von der Bilanziervorrichtung 104 übermittelte Ultrafiltrationswert geregelt wird. Liegt etwa der übermittelte Wert der Ultrafiltrationsrate über einem entsprechenden Sollwert, so ist die Blutpumpe 115 zu drosseln, liegt die Ultrafiltrationsrate unter ihrem Sollwert, so wird die Blutpumpe 115 beschleunigt.

Eine Kombination der Regelstrategien ist möglich, etwa in der Art, dass in einer inneren Regelschleife zunächst die Blutpumpe 115 konstant betrieben wird und die Dialysierflüssigkeitspumpe 107 gesteuert wird. Erst wenn ein Grenzwert für die Dialysierflüssigkeitspumpe erreicht wird, wird die Blutpumpe 115 entsprechend angesteuert.

Eine alternative Kombination läge darin, in einer Art innerer Regelschleife die Dialysierflüssigkeitspumpe konstant zu betreiben und die Blutpumpe 115 zu steuern. Wenn ein Grenzwert für einen Betriebsparameter der Blutpumpe 115 erreicht wird, wird zusätzlich die Dialysierflüssigkeitspumpe angesteuert.

Die Regelung der Ultrafiltration kann so erfolgen, dass für die Ultrafiltrationsrate ein bestimmter Wert vorgegeben wird. Alternativ kann für das während der Blutbehandlung zu entziehende Ultrafiltrationsvolumen ein bestimmtes Ultrafiltrationsprofil vorgegeben werden.

Der vorgegebene Wert der Ultrafiltrationsrate kann ein konstanter oder sich kontinuierlich ändernder Wert für die Ultrafiltrationsrate sein.

Alternativ kann für die Ultrafiltrationsrate oder das Ultrafiltrationsvolumen ein Profil vorgegeben werden, bei dem sich Intervalle mit einer positiven Ultrafiltrationsrate und Intervalle mit einer negativen Ultrafiltrationsrate abwechseln. Auf diese Weise kann ein sogenannter push/ pull Modus erreicht werden, bei dem Ablagerungen von der Dialysatormembran abgelöst oder ein Anlagern von Substanzen an die Dialysatormembran vermindert oder verhindert wird. Dadurch wird die Durchlässigkeit der Dialysatormembran sowie die entsprechende Reinigungsleistung (Clearance) für Mittelmoleküle verbessert. Die vorliegende Anordnung erreicht dies ohne zusätzlichen apparativen Aufwand, etwa: ohne eine zusätzliche Pumpe für das Aufbringen oszilierender Druckpulse.

Die Regelung der Ultrafiltration kann in diesem Fall analog zu den oben für die Ultrafiltrationsrate beschriebenen Regelstrategien erfolgen, wobei an die Stelle eines Abgleichs mit dem Sollwert der Ultrafiltrationsrate ein entsprechender Abgleich mit einem Ultrafiltrationsprofil erfolgt.

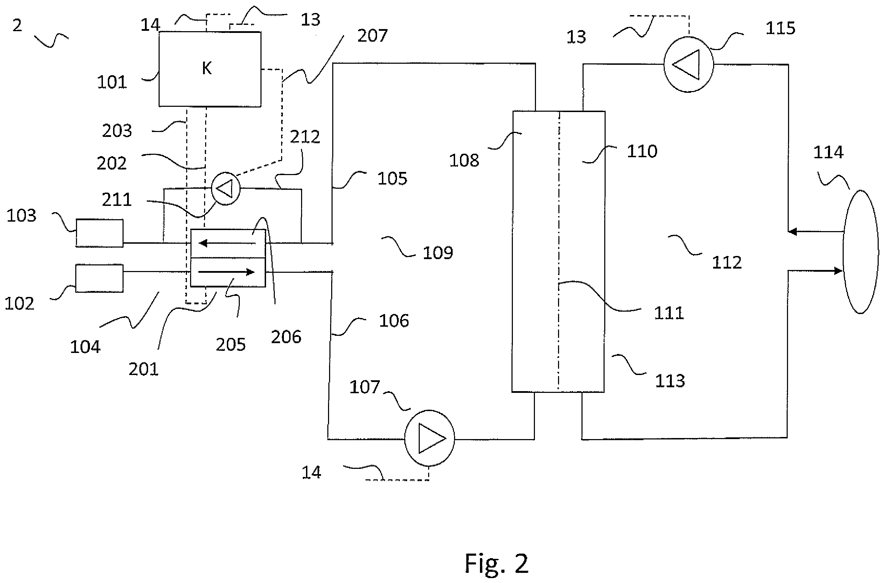

Figur 2 zeigt schematisch ein weiteres Dialysegerät mit einer weiteren Vorrichtung zur Regelung der Ultrafiltration. Das in Figur 2 dargestellte Dialysegerät entspricht im Wesentlichen dem Aufbau des Ultrafiltrationsgeräts der Figur 1. Auf die Beschreibung der entsprechenden Elemente wird Bezug genommen an Stelle einer Wiederholung. Die Darstellung des Ultrafiltrationsgeräts unterscheidet sich im Wesentlichen durch die Ausführung der Bilanziervorrichtung 104, die im Folgenden genauer beschrieben wird.

Die Bilanziervorrichtung 104 umfasst die zu einem Differenzstromsensor 201 verbundenen Flussmesszellen 205 und 206, wobei die Flussmesszelle 205 stromauf der Dialysierflüssigkeitskammer 108 und die Flussmesszelle 206 stromab der Dialysierflüssigkeitskammer 108 im Dialysierflüssigkeitskreislauf 109 liegt.

Eine Ultrafiltrationspumpe 211 liegt in einem zur Flussmesszelle 206 parallelen Flüssigkeitsweg 212, in dem der Flüssigkeitstransport durch die Ultrafiltrationspumpe 211 kontrolliert wird.

Der Differenzstromsensor 201 ermittelt ein Messwertpaar bestehend aus einen separaten Messwert für jede Flussmesszelle 205, 206 der die Strömungsgeschwindigkeit der Flüssigkeit durch die jeweilige Flussmesszelle anzeigt. Das Messwertpaar wird bevorzugt ein oder mehrmals pro Sekunde ermittelt und über Messleitungen 202 und 203 zu der Steuer- und Regeleinheit 101 übertragen. Die Steuer- und Regeleinheit 101 ordnet jedem Messwertpaar ein Volumenstrompaar zu, wobei eine Abbildung von einem Messwert auf einen Volumenstrom verwendet werden kann, die auf einer zuvor durchgeführten Kalibrierung beruht. Alternativ könnte auch eine Abbildung auf einen Massenstrom erfolgen. Die Steuer- und Regeleinheit 101 leitet aus dem ermittelten Volumenstrompaar ein Steuersignal für die Pumpe 211 ab, etwa so, dass die Pumpe 211 derart betrieben wird, dass der Volumenstrom durch beide Flussmesszellen 205 und 206 des Differenzstromsensors übereinstimmt. Beispielsweise bildet die Steuer- und Regeleinheit 101 ein Differenzsignal aus beiden Volumenströmen des Volumenstrompaares und verändert die Flussrate der Ultrafiltrationspumpe 211 durch Erhöhung oder Verringerung je nach Vorzeichen des Differenzsignal in geeigneter Weise so, dass das Differenzsignal zu null verschwindet. Ist der Fluss durch die Flussmesszelle 205 kleiner als der Fluss durch die Flussmesszelle 206, so ergibt sich für die Differenz der Messwerte von Flussmesszelle 206 und Flussmesszelle 205 ein positiver Wert. Die Steuer- und Regeleinheit 101 kann nun das Steuersignal für Ultrafiltrationspumpe 211 so verändern, dass sich die Flussrate durch Ultrafiltrationspumpe 211 erhöht und der Fluss durch Flussmesszelle 206 bei unverändertem Fluss im Abfluss des Dialysators soweit verringert bis sich der gleiche Fluss wie durch Flussmesszelle 205 einstellt. Die Flussrate durch die Ultrafiltrationspumpe 211 zeigt dann den Differenzfluss zwischen dem aus der Dialysierflüssigkeitskammer austretenden Dialysierflüssigkeitsfluss und dem in die Dialysierflüssigkeitskammer eintretenden Dialysierflüssigkeitsfluss an. Die Flussrate durch die Ultrafiltrationspumpe 211 ist dann ein Maß für die Menge des im Dialysator 113 entzogenen Ultrafiltrats.

In einer Ausführungsform wird die Flussrate durch die Ultrafiltrationspumpe 211 auf einen vorbestimmten Wert eingestellt, und die Steuerung der Blutpumpe 115 sowie der Dialysierflüssigkeitspumpe 107 erfolgt wie oben beschrieben so, dass der im Differenzstromsensor 201 gemessene Differenzstrom eine vorbestimmte Bedingung erfüllt, etwa: zu null verschwindet.

Hierbei ist die Flussrate durch die Ultrafiltrationspumpe 211 ein Maß für die Flüssigkeitsbilanz zwischen dem Zufluss zu dem Dialysator 113 und dem Abfluss aus dem Dialysator 113, d.h. für die Menge des im Dialysator 113 entzogenen Ultrafiltrats.

Die Bilanziereinheit 104 als Ganzes wirkt wie ein passives Bauelement und bewirkt keine aktive Steuerung oder Regelung der Flüssigkeitsbilanz zwischen der aus dem Dialysator abfließenden und der in den Dialysator 113 zufließenden Dialysierflüssigkeit.

Das Verschwinden des Differenzsignals kann sich auf einen Differenzfluss zu einem bestimmten Zeitpunkt beziehen oder auf das Verschwinden eines Integrals eines Differenzflusses.

In einer weiteren Ausgestaltung kann auf die Zuordnung des Messwertpaars zu einem Volumen- oder Massenstrom verzichtet werden, wenn die Differenz der Messwerte bei gleichem Volumenstrom durch beide Kanäle bekannt ist. In diesem Fall bildet die Steuer- und Regeleinheit 101 die Differenz aus beiden Messwerten und verändert die Flussrate der Ultrafiltrationspumpe 211 durch Erhöhung oder Verringerung der Differenz in geeigneter Weise solange bis die Differenz der vorbekannten Differenz bei gleichem Volumenstrom entspricht.

Der Differenzstromsensor 201 kann vorteilhaft nach dem Magentisch-Induktiven Prinzip funktionieren. Dabei weisen die beiden Flussmesszellen 205, 206, die im Gegenstrom durchströmt werden, einen vorzugsweise rechteckigen Querschnitt auf und werden rechtwinkelig zu einem Magnetfeld angeordnet. Das Magnetfeld wird dabei von der Steuerung des Differenzstromsensors 201 eingestellt und ist so beschaffen, dass durch beide Flussmesszellen 205, 206 ein homogenes Feld vorherrscht. Dies wird beispielsweise dadurch erreicht, dass die Kanäle der Flussmesszellen 205, 206 benachbart zueinander in einem Magnetfeld angeordnet sind. In jedem Kanal ist gegenüberliegend und im rechten Winkel zum Magnetfeld und zur Flussrichtung im jeweiligen Kanal eine Elektrode an derjenigen inneren Kanalwand angebracht, die sich entlang des Magnetfeldes erstreckt. Strömt Flüssigkeit durch den Kanal, so wird durch das Magnetfeld eine Ladungstrennung der in der Flüssigkeit vorliegenden Ionen bewirkt, so dass an den Elektroden eine elektrische Spannung vorliegt. Diese Spannung ist proportional zu der Strömungsgeschwindigkeit und abhängig von der Magnetfeldstärke. Ist das Magnetfeld in den beiden Flussmesszellen 205 und 206 gleich groß, so fällt bei der Bildung eines Differenzsignals aus beiden Kanälen die Magnetfeldstärkeabhängigkeit für das relative Differenzflusssignal vorteilhaft weg.

Mit anderen Worten: ein Verschwinden des Differenzsignals zeigt unabhängig von der absoluten Größe des Magnetfelds in den Flussmesszellen 205 und 206 an, dass der Fluss durch die Flussmesszelle 205 und der Fluss durch die Flussmesszelle 206 gleich groß sind.

In der Ausführungsform, bei der für die Ultrafiltrationsrate oder für das Ultrafiltrationsvolumen ein Profil vorgegeben werden, bei dem sich Intervalle mit einer positiven Ultrafiltrationsrate und Intervalle mit einer negativen Ultrafiltrationsrate abwechseln, ist die vorbestimmte Bedingung vorteilhaft dann erfüllt, wenn das Integral der Ultrafiltrationsrate und/ oder des Differenzsignals zu Null verschwindet.

Die Ultrafiltrationspumpe 211 ist bevorzugt aus der Gruppe der Verdrängerpumpen gewählt, bevorzugter eine Membran-, Schlauchrollen-, Kolben- oder Zahnradpumpe oder jede andere Pumpe die es erlaubt, die geförderte Flüssigkeitsmenge zu ermitteln. Beispielsweise kann mit der Schlauchrollenpumpe das geförderte Volumen durch das Pumpschlauchvolumen und dem Drehwinkel des Rotors der Schlauchrollenpumpe mit guter Genauigkeit mit bekannten Verfahren bestimmt werden. Auch für andere Pumpen aus der Gruppe der Verdrängerpumpen sind entsprechende Verfahren zur Bestimmung der geförderten Flüssigkeitsmenge aus dem Stand der Technik bekannt.

Vorteilhaft ist hierbei, dass die zu messende Flüssigkeitsmenge der Ultrafiltratmenge entspricht. Diese Menge liegt typischerweise bei 3-5l je Dialysebehandlung oder Tag, wohingegen die Menge an Dialysat, die durch den Flusssensor strömt, ein Vielfaches, typischerweise 60-240l davon beträgt. Im Einklang mit der vorliegenden Offenbarung ist es daher vorteilhaft möglich, Messgeräte oder Messverfahren für den Differenzfluss einzusetzen, die eine wesentlich geringere Toleranz aufweisen müssen, als dies Messverfahren müssten, die die abfließende und die zufließende Menge an Dialysat getrennt erfassen, und erst anschließend eine Differenzbildung vornehmen.

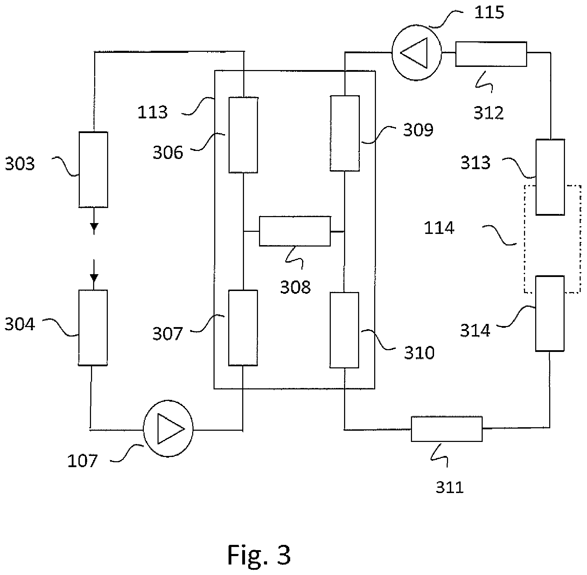

Figur 3 zeigt ein Ersatzschaltbild der in Figur 1 dargestellten Dialysevorrichtung mit der Dialysierflüssigkeitspumpe 107, der Blutpumpe 115, sowie dem Dialysator 113, wobei die Flusswiderstände im Dialysierflüssigkeitskreislauf, im Blutkreislauf sowie im Dialysator als Widerstände eines elektrischen Ersatzschaltbildes dargestellt sind. Im Einzelnen sind im extrakorporalen Blutkreislauf ein arterieller Nadelwiderstand 313, ein arterieller Leitungswiderstand 312, ein venöser Nadelwiderstand 314, ein venöser Leitungswiderstand 311, sowie im Dialysator 113 ein arterieller Filter-Längswiderstand 309, und ein venöser Filter-Längswiderstand 310 dargestellt. Im Dialysierflüssigkeitskreislauf werden die Flusswiderstände durch einen eingangsseitigen Flusswiderstand des Dialysators 307, einen ausgangsseitigen Flusswiderstand des Dialysators 306, einen Flusswiderstand 304 auf der Dialysateingangsseite des Dialysierflüssigkeitskreislaufs, sowie einen Flusswiderstand 303 auf der Dialysatausgangsseite des Dialysierflüssigkeitskreislaufs modelliert. Die Membran im Dialysator wird durch einen Transmembranwiderstand 308 modelliert.

Tabelle 1

| Bezeichnung des Widerstandes | Bezugszeich en | Formelzeichen |
|---|---|---|
| arterieller Nadelwiderstand im extrakorporalen Blutkreislauf | 313 | $R_{aN}$ |
| arterieller Leitungswiderstand im extrakorporalen Blutkreislauf | 312 | $R_{aL}$ |
| venöser Nadelwiderstand im extrakorporalen Blutkreislauf | 314 | $R_{vN}$ |
| venöser Leitungswiderstand im extrakorporalen Blutkreislauf | 311 | $R_{aN}$ |
| arterieller Filter-Längswiderstand | 309 | $R_{aF}$ |
| venöser Filter-Längswiderstand | 310 | $R_{vF}$ |
| eingangsseitiger Flusswiderstand im Dialysierflüssigkeitskreislauf | 304 | $R_{Din}$ |
| ausgangsseitiger Flusswiderstand im Dialysierflüssigkeitskreislauf | 303 | $R_{Dout}$ |
| eingangsseitiger Flusswiderstand des Dialysators | 307 | $R_{DFin}$ |
| ausgangsseitiger Flusswiderstand des Dialysators | 306 | $R_{DFout}$ |
| Transmembranwiderstand | 308 | $R_{TM}$ |

Für die unten angegebene Ableitung für eine Dimensionierung der Widerstände im Dialysierflüssigkeitskreislauf, im extrakorporalen Blutkreislauf sowie des Transmembranwiderstandes sind die Bezeichnungen der einzelnen Widerstände, ihre Bezugszeichen, sowie die in der Ableitung verwendeten Formelzeichen in der Tabelle 1 wiedergegeben.

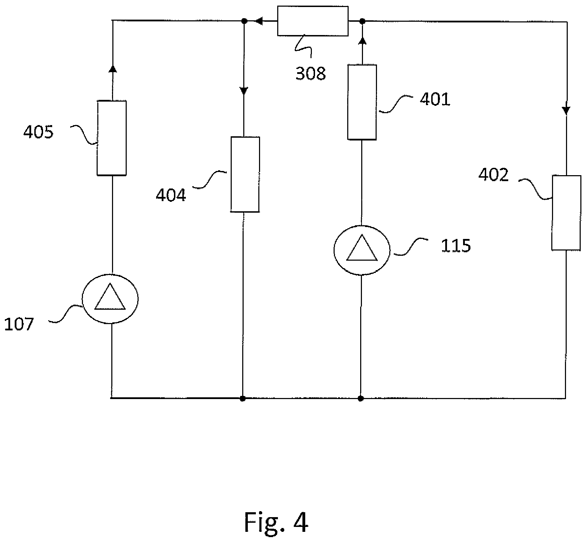

Ein vereinfachtes Ersatzschaltbild des in Figur 3 dargestellten elektrischen Ersatzschaltbildes zeigt Figur 4. In dem in Figur 4 dargestellten vereinfachten Ersatzschaltbild sind der arterieller Nadelwiderstand im extrakorporalen Blutkreislauf (Formelzeichen: $R_{aN}$), der arterielle Leitungswiderstand im extrakorporalen Blutkreislauf (Formelzeichen: $R_{aL}$) sowie der arterielle Filter-Längswiderstand (Formelzeichen: $R_{aF}$) zu einem arteriellen Gesamtwiderstand 401 (Formelzeichen: $R_a$) zusammengefasst:

$$R_a = R_{aN} + R_{aL} + R_{aF} \qquad \text{(Gleichung 1)}$$

Ebenso können der venöser Nadelwiderstand im extrakorporalen Blutkreislauf (Formelzeichen: $R_{vN}$) der venöse Leitungswiderstand (Formelzeichen: $R_{vL}$) im extrakorporalen Blutkreislauf, sowie der venöse Filter-Längswiderstand (Formelzeichen: $R_{vF}$) zu einem venösen Gesamtwiderstand 402 (Formelzeichen: $R_v$) zusammengefasst werden:

$$R_v = R_{vN} + R_{vL} + R_{vF} \qquad \text{(Gleichung 2)}$$

Eine entsprechende Zusammenfassung der Widerstände im Dialysierflüssigkeitskreislauf ergibt Folgendes. Der eingangsseitige Flusswiderstand im Dialysierflüssigkeitskreislauf 304 (Formelzeichen: $R_{Din}$) und der eingangsseitige Flusswiderstand 307 des Dialysators (Formelzeichen: $R_{DFin}$) können zu einem Eingangswiderstand 405 (Formelzeichen: $R_{in}$) zusammengefasst werden:

$$R_{in} = R_{Din} + R_{DFin} \qquad \text{(Gleichung 3)}$$

Der ausgangsseitige Flusswiderstand im Dialysierflüssigkeitskreislauf 303 (Formelzeichen: $R_{Dout}$) und der ausgangsseitige Flusswiderstand des Dialysators 306 (Formelzeichen: $R_{DFout}$) können zu einem Ausgangswiderstand 404 (Formelzeichen: $R_{out}$) zusammengefasst werden:

$$R_{out} = R_{Dout} + R_{DFout} \qquad \text{(Gleichung 4)}.$$

Die Bezeichnungen der in Figur 4 dargestellten Widerstände, ihrer Bezugszeichen, sowie die in der Ableitung verwendeten Formelzeichen sind in der folgenden Tabelle 2 widergegeben.

Tabelle 2

| Bezeichnung des Widerstandes | Bezugszeichen | Formelzeichen |
|---|---|---|
| arterieller Gesamtwiderstand | 401 | $R_a$ |
| venöser Gesamtwiderstand | 402 | $R_v$ |
| Eingangswiderstand (im Dialysierflüssigkeitskreislauf) | 405 | $R_{in}$ |
| Ausgangswiderstand (im Dialysierflüssigkeitskreislauf) | 404 | $R_{out}$ |
| Transmembranwiderstand | 308 | $R_{TM}$ |

Die Blutpumpe und die Dialysierflüssigkeitspumpe können als Stromquelle oder als Spannungsquelle modelliert werden, wobei die geeignete Modellierung von der Bauart der Pumpe beeinflusst wird. So ist bei der Verwendung einer Verdrängerpumpen, etwa einer Membran-, Schlauchrollen-, Kolben- oder Zahnradpumpe als Dialysierflüssigkeitspumpe die Modellierung der Dialysierflüssigkeitspumpe als Stromquelle möglich. Entsprechendes gilt, wenn die Blutpumpe als Verdrängerpumpe, etwa als Schlauchrollenpumpe ausgeführt ist. Eine druckkonstante Pumpe wie etwa eine Impellerpumpe wird vorzugsweise als Spannungquelle modelliert. Werden die Blutpumpe oder die Dialysierflüssigkeitspumpe als nicht-ideale Spannungs- oder Stromquelle mit entsprechenden Innenwiderständen modeliert, so müssen die jeweiligen Innenwiderstände den Widerständen im Dialysierflüssigkeitskreislauf beziehungsweise im extrakorporalen Blutkreislauf noch zugeschlagen werden. So muss etwa bei der Modellierung der Dialysierflüssigkeitspumpe als nicht-ideale Spannungsquelle der Strömungwiderstand der Dialysierflüssigkeitspumpe in den Eingangswiderstand 405 aufgenommen werden. Entsprechendes gilt für den extrakorporalen Blutkreislauf. Dem Fachmann sind die entsprechenden hierzu erforderlichen Überlegungen bekannt. Dem Fachmann ist ebenfalls bekannt wie Ersatzschaltbilder, bei denen nicht- ideale Spannungsquellen modelliert werden in entsprechende Ersatzschaltbilder mit nicht-idealen Stromquellen umzuwandeln sind. Bei der Dimensionierung der Widerstände im extrakorporalen Blutkreislauf und im Dialysierflüssigkeitskreislauf sowie bei der Dimensionierung der Innenwiderstände der beteiligten Pumpen, und beim Ansteuern der beteiligten Pumpen zum Erzielen einer gewünschten Ultrafiltrationsrate können folgende Überlegungen hilfreich sein. Wenn für die Ultrafiltrationsrate ein entsprechender Strom $I_{UF}$ im elektrischen Ersatzschaltbild angesetzt wird, so kann im Fall der Modellierung der Pumpen als Spannungsquellen, wenn die Dialysierflüssigkeitspumpe mit einer Spannungsquelle der Spannung $U_D$ und die Blutpumpe mit einer Spannungsquelle der Spannung $U_B$ modelliert wird, folgende Formel für den die Ultrafiltrationsrate angegeben werden:

$$I_{UF} = \frac{\dfrac{U_B \cdot R_V}{R_a + R_V} - \dfrac{U_D \cdot R_{out}}{R_{in} + R_{in}}}{\dfrac{R_a \cdot R_v}{R_a + R_v} + \dfrac{R_{in} \cdot R_{out}}{R_{in} + R_{out}} + R_{TM}} \qquad \text{(Gleichung 5)}$$

Im Fall der Modellierung der Pumpen als Stromquellen kann, wenn die Dialysierflüssigkeitspumpe mit einer Stromquelle des Stroms $I_D$ und die Blutpumpe mit einer Stromquelle des Stroms $I_B$ modelliert wird, folgende Formel für die Ultrafiltrationsrate angegeben werden:

$$I_{UF} = \frac{I_B \cdot R_V - I_D R_D}{R_v + R_{out} + R_{TM}} \qquad \text{(Gleichung 6)}$$

Für die Dimensionierung des Ausgangswiderstandes 404 (Formelzeichen $R_{out}$) ist folgende Überlegung hilfreich. Die Umstellung der Formel 6 für den zum Erzielen einer bestimmten Ultrafiltrationsrate erforderlichen Ausgangswiderstand 404 (Formelzeichen $R_{out}$) ergibt folgende Beziehung:

$$R_{out} = \frac{R_v \cdot (I_B - I_{UF}) - R_{TM} I_{UF}}{I_D - I_{UF}} \qquad \text{(Gleichung 7)}$$

Die Formel 7 zeigt, dass ein zu hoher Transmembranwiderstand $R_{TM}$ einen ungünsigen Einfluss auf die Dimensionierung des Ausgangswiderstands $R_{out}$ im Dialysierflüssigkeitskreislauf hat. Der Transmembranwiderstand $R_{TM}$ sollte daher möglichst klein gewählt werden, etwa als Filter mit einem hohen spezifischen Durchgangskoeffizienten ("high Cut-off Filter") oder als Filter mit einer ausreichend großen effektiven Filterfläche. Dabei ist zu berücksichtigen, dass der Transmembranwiderstand $R_{TM}$ sich ebenso wie der venöse Filter-Längswiderstand $R_{vF}$ im Verlauf der extrakorporalen Blutbehandlung zunehmen werden. Die Zunahme des venösen Filter-Längswiderstandes $R_{vF}$ im extrakorporalen Blutkreislauf beruht typischerweise auf der Zunahme des Hämatokrits im Laufe der Blutbehandlung, der sogenannten Hämokonzentration, sowie auf einem möglichen Entstehen von Engstellen im extrakorporalen Blutkreislauf. Eine Zunahme des Transmembranwiderstandes $R_{TM}$ im Laufe einer Blutbehandlung entsteht häufig durch Ablagerungen an der Dialysatormembran. Diese Effekte, die sich regelmäßig im Laufe einer Blutbehandlung einstellen, müssen bei der Dimensionierung der Widerstände im extrakorporalen Blutkreislauf und im Dialysierflüssigkeitskreislauf berücksichtigt werden.

Folgendes Zahlenbeispiel kann einen Anhaltspunkt für eine mögliche Dimensionierung der beteiligten Flüsse geben, d.h. für den Blutflusse $I_B$, wird von einem Minimalwert $I_{Bmin}$ = 60 ml/min und einem Maximalwert $I_{bmax}$ = 300 ml/min ausgegangen wird, für die Ultrafiltrationsrate $I_{UF}$ von einem Maximalwert $I_{UFmax}$ = $I_B$/ 10, also etwa 20 ml/min und einem Minimalwert $I_{UFmin}$ = 0 ml/min sowie für die Dialysierflüssigkeitsrate $I_D$ von einem Minimalwert $I_{Dmin}$ = $I_B$/ 3 und einem Maximalwert $I_{Dmax}$ = 200 ml/min.

Der Blutfluss $I_B$ ist dabei als der Fluss durch die Blutpumpe 115 gesetzt, der Dialysierflüssigkeitsrate $I_D$ entspricht ist dem Fluss durch die Dialysierflüssigkeitspumpe 107 und die Ultrafiltrationsrate $I_{UF}$ entspricht dem Fluss durch den Transmembranwiderstand 308.

Für die Dialysierflüssigkeitsrate ID gilt allgemein folgende Beziehung:

$$I_D = \frac{I_B \cdot R_V - I_{UF} \cdot (R_V + R_{out} + R_{TM})}{R_{out}} \qquad \text{(Gleichung 8)}$$

Grundsätzlich ergibt sich bei einem vorgegebenen Blutfluss $I_B$, dass eine maximale Ultrafiltration dann erzielt wird, wenn die Dialysierflüssigkeitsrate $I_D$ minimal ist. Nach einer Umstellung von Gleichung 8 ergibt sich so folgende Beziehung:

$$I_{D\min} \leq \frac{R_V}{R_{out}} \cdot (I_B - I_{UF}) - \left(1 + \frac{R_{TM}}{R_{out}}\right) I_{UF} \qquad \text{(Gleichung 9)}$$

Setzt man als alternative Zahlenwerte für den minimalen Blutfluss $I_B$ = 50 ml/min ein und für die Ultrafiltrationsrate $I_{UF}$ = 5 ml/min, so ergibt sich die folgende Beziehung, die die Größen der Widerstände $R_v$, $R_{out}$ und $R_{TM}$ zu einander in eine Beziehung setzt:

$$20 \leq \frac{R_V}{R_{out}} \cdot (50 - 5) - \left(1 + \frac{R_{TM}}{R_{out}}\right) 5 \qquad \text{(Gleichung 10)}$$

oder:

$$25 \leq 45 \cdot \frac{R_V}{R_{out}} - 5 \cdot \frac{R_{TM}}{R_{out}} \qquad\qquad \text{(Gleichung 11)}$$

Ausgehend von einem bestimmten Verhältnis zwischen dem Transmembranwiderstand $R_{TM}$ und dem Ausgangswiderstand im Dialysierflüssigkeitskreislauf, ergeben sich die folgenden Beziehungen für die Dimensionierung des Ausgangswiderstands $R_{out}$ im Dialysierflüssigkeitskreislauf in Bezug zu dem venösen Gesamtwiderstand $R_V$, wobei aufsteigende Werte für den Transmembranwiderstand $R_{TM}$ angegeben sind, die die oben angesprochenen Effekte eines Anstiegs über den Behandlungsverlauf wiederspiegeln.

$$R_{TM} = R_{out} / 2 \qquad -> \qquad R_{out} \leq \frac{90}{55} R_V$$

$$R_{TM} = R_{out} \qquad -> \qquad R_{out} \leq \frac{45}{30} R_V$$

$$R_{TM} = 2 \cdot R_{out} \qquad -> \qquad R_{out} \leq \frac{45}{35} R_V$$

$$R_{TM} = 4 \cdot R_{out} \qquad -> \qquad R_{out} \leq \frac{45}{45} R_V$$

Diese Beispielrechnung zeigt, dass der Ausgangswiderstand $R_{out}$ umso kleiner gewählt werden muss, wenn der Transmembranwiderstand $R_{TM}$ größer ist. Wie bereits oben angesprochen ist für die Auslegung der Flusswiderstände im Dialysierflüssigkeitskreislauf und im extrakorporalen Blutkreislauf ein zu hoher Transmembranwiderstand $R_{TM}$ nachteilig. Nimmt man beispielsweise an, das der Transmembranwiderstand $R_{TM}$ als Maximalwert das Vierfache des Ausgangswiderstands $R_{out}$ im Dialysierflüssigkeitskreislauf annehmen kann, so ergibt das oben angeführte Zahlenbeispiel die einfache Forderung, dass $R_{out}$ kleiner als $R_V$ sein muss. Für die Auslegung des Ausgangswiderstands $R_{out}$ im Dialysierflüssigkeitskreislauf und des venösen Gesamtwiderstandes $R_V$ ist es ausreichend, den venösen Gesamtwiderstand $R_V$ zu Beginn der Blutbehandlung anzusetzen, da mit einem während der Blutbehandlung ansteigenden venösen Gesamtwiderstand die Gleichung 9 umso eher erfüllt sein wird.

Für die nachfolgende beispielhafte Angabe von Werten für die Rate des Dialysierflüssigkeitsflusses $I_D$, des Blutflusses $I_B$, sowie der Ultrafiltrationsrate $I_{UF}$ wird davon ausgegangen, dass zu Beginn der Behandlung der Wert des Ausgangswiderstandes $R_{out}$ dem venösen Gesamtwiderstand $R_V$ entspricht und dass der Transmembranwiderstand $R_{TM}$ zu Beginn der Behandlung dem Ausgangswiderstandes $R_{out}$ entspricht.

Bei einem Blutfluss $I_B$ = 200 ml/min, wird ausgehend von Gleichung 9 die maximale Ultrafiltrationsrate $I_{UF}$ = 20 ml/min bei einer Dialysierflüssigkeitsrate

$$I_D = (200 - 20) ml / \min - 2 \cdot 20 ml / \min = 140 ml / \min$$

erzielt.

Die minimale Ultrafiltrationsrate $I_{UF}$ = 0 ml entspricht einer Dialysierflüssigkeitsrate

$$I_D = (200 - 0) ml / \min - 2 \cdot 0 ml / \min = 200 ml / \min$$

Beide Werte liegen in einem zulässigen, akzeptablen oder bevorzugten Bereich der Dialysierflüssigkeitsrate $I_D \leq$ 200 ml/min.

Für das folgende Zahlenbeispiel wird davon ausgegangen, dass im weiteren Verlauf der Blutbehandlung der Transmembranwiederstand $R_{TM}$ in Folge der oben angesprochenen Effekte auf den vierfachen Wert ansteigt. Um eine maximale Ultrafiltrationsrate $I_{UF}$ = 20 ml/min zu erzielen, wäre eine Drosselung der Dialysierflüssigkeitsrate auf

$$I_D = (200-20)ml/\min - 5\cdot 20ml/\min = 80ml/\min$$

erforderlich.

Für die minimale Ultrafiltrationsrate $I_{UF}$ = 0 ml wäre nach wie vor eine Dialysierflüssigkeitsrate

$$I_D = (200-0)ml/\min - 5\cdot 0ml/\min = 200ml/\min \text{ einzustellen.}$$

Nimmt man an, dass sich in Folge der oben angesprochenen Effekte der venöse Gesamtwiderstand $R_v$ im Laufe der Blutbehandlung verdoppelt, so ergibt sich für eine maximale zu erzielende Ultrafiltrationsrate von 20 ml/min ein erforderlicher Dialysierflüssigkeitsfluss von

$$I_D = 2\cdot(200-20)ml/\min - 5\cdot 20ml/\min = 260ml/\min .$$

Für eine minimale Ultrafiltrationsrate $I_{UF}$ = 0 ml ergibt sich ein Diylsierflüssigkeitsfluss von

$$I_D = 2\cdot(200-0)ml/\min - 5\cdot 0ml/\min = 400ml/\min .$$

Um in diesem Fall die Ultrafiltrationsrate über den gesamten Bereich von 0 bis 20 ml/min regeln zu können, besteht die Möglichkeit, den als zulässig, akzeptabel oder bevorzugt angesehenen Bereich des Dialysierflüssigkeitsflusses zu erweitern oder den Blutfluss $I_B$ zu verringern. So würde etwa bei einem maximal zulässigen Dialysierflüssigkeitsfluss $I_D$ = 200 ml/min, eine maximale Ultrafiltrationsrate $I_{UF}$ = 20 ml/min bei einem Blutfluss $I_B$ = 170 ml/min erzielt, und eine minimale Ultrafiltrationsrate $I_{UF}$ = 0 ml/min bei einem Blutfluss $I_B$ = 100 ml/min.

Das folgende Rechenbeispiel soll verdeutlichen, wie vorteilhaft es ist, den Transmembranwiderstand $R_{TM}$ gering zu halten. Wenn man etwa durch die Dimensionierung des Dialystors und des Dialysierflüssigkeitskreislaufs sicherstellt, dass der Transmembranwiderstand $R_{TM}$ maximal dem Ausgangswiderstand $R_{out}$ im Dialysierflüssigkeitskreislauf entspricht, und legt man den Dialysierflüssigkeitskreislauf und den extrakorporalen Blutkreislauf so aus, dass der Ausgangswiderstand $R_{out}$ im Dialysierflüssigkeitskreislauf zu dem venösen Gesamtwiderstand in einem Verhältnis $R_{out}$ = 3/2 $R_v$ ist, so ergibt sich bei einem Blutfluss $I_B$ = 200 ml/min eine maximale Ultrafiltrationsrate von 20 ml/min bei einer Dialysierflüssigkeitsrate

$$I_D = 4/3\cdot(200-20)ml/\min - 2\cdot 20ml/\min = 200ml/\min$$

und bei demselben Blutfluss $I_B$ eine minimale Ultrafiltrationsrate von 0 ml/min bei einer Dialysierflüssigkeitsrate

$$I_D = 4/3\cdot(200-0)ml/\min - 2\cdot 0ml/\min = 267ml/\min .$$

Der Bereich einer Ultrafiltrationsrate $I_{UF}$ von 0 ml/ min bis 20 ml/min kann somit mit einer geringeren Variation der Dialysierflüssigkeitsrate $I_D$ angesteuert werden.

**Patentansprüche**

1. Vorrichtung (1, 2) zur Steuerung oder Regelung einer Ultrafiltration bei einer Dialysebehandlung,
   bei der zu ultrafiltrierendes Blut in einem extrakorporalen Blutkreislauf (112) eine Blutkammer (110) eines durch eine semipermeable Membran (111) in die Blutkammer (110) und eine Dialysierflüssigkeitskammer (108) unterteilten Dialysators (113) durchströmt und Dialysierflüssigkeit in einem Dialysierflüssigkeitskreislauf (109) die Dialysierflüssigkeitskammer (108) des Dialysators (113) durchströmt,
   mit einer Blutpumpe (115) zur Erzeugung eines Blutflusses in dem extrakorporalen Blutkreislauf (112), mit einer Dialysierflüssigkeitstpumpe (107) zur Erzeugung eines Dialysierflüssigkeitsflusses in dem Dialysierflüssigkeitskreislauf (109),
mit einer Bilanziervorrichtung (104) zum Aufstellen einer Flüssigkeitsbilanz im Dialysierflüssigkeitskreislauf zwischen einem Zustrom (106) und einem Abfluss (105) der Dialysierflüssigkeitskammer (113) als Maß für die Ultrafiltration, **gekennzeichnet durch**:

einer Regeleinheit (101) zum Regeln der Blutpumpe (115) und/oder der Diaylsierflüssigkeitspumpe (107) so dass eine vorbestimmte Ultrafiltration erzielt wird ohne weitere aktive Steuerung oder Regelung des aus dem Dialysator abfließenden Dialysierflüssigkeitsflusses, wobei die Dialysierflüssigkeitspumpe (107) in einer Zuleitung zu der Dialysierflüssigkeitskammer (113) angeordnet ist und keine weitere aktive Steuerung oder Regelung des Dialysierflüssigkeitsflusses stromab der Dialysierflüssigkeitskammer (113) stattfindet.

2. Vorrichtung (1, 2) zur Steuerung oder Regelung einer Ultrafiltration nach Anspruch 1, wobei die Regeleinheit (101) angepasst ist, eine Ultrafiltrationsrate und/oder ein während eines Behandlungsverlaufs zu entziehendes Ultrafiltrationsvolumen vorzugegeben.

3. Vorrichtung (1, 2) nach einem der vorangehenden Ansprüche, wobei die Blutpumpe in einer Zuleitung zu der Blutkammer angeordnet ist.

4. Vorrichtung (1, 2) nach einem der vorangehenden Ansprüche, wobei für den Blutfluss ein vorbestimmter Wert einstellbar ist und die Regeleinheit angepasst ist zur Regelung des Dialysierflüssigkeitsflusses.

5. Vorrichtung (1, 2) nach einem der vorangehenden Ansprüche, wobei für den Dialysierflüssigkeitsfluss ein vorbestimmter Wert einstellbar ist und die Regeleinheit angepasst ist zur Regelung des Blutflusses.

6. Vorrichtung (1, 2) nach einem der vorangehenden Ansprüche, wobei durch die Regeleinheit (101) ein Profil für die Ultrafiltrationsrate vorgebbar ist, bei dem sich Intervalle mit einer positiven Ultrafiltrationsrate und Intervalle mit einer negativen Ultrafiltrationsrate abwechseln.

7. Vorrichtung (2) nach einem der vorangehenden Ansprüche, wobei die Bilanziervorrichtung eine Differenzflussmesseinheit (104) zum Messen des Differenzflusses zwischen einem Fluss in dem Zustrom zur Dialysierflüssigkeitskammer (108) und dem Abfluss aus der Dialysierflüssigkeitskammer, eine Verzweigung von dem Zufluss oder dem Abfluss zur Abzweigung von Dialysierflüssigkeit von dem Zufluss oder dem Abfluss in einen weiteren Flussweg (212), sowie eine Einrichtung zur Einstellung der Flussmenge (211) in dem Zufluss, in dem Abfluss und/ oder in dem weiteren Flussweg, aufweist, die derart ansteuerbar ist, dass der gemessene Differenzfluss eine vorbestimmte Bedingung erfüllt, und mit einer Einrichtung (211) zur Ermittlung der Flussmenge in dem weiteren Flussweg als Maß der Flüssigkeitsbilanz.

8. Vorrichtung (2) nach Anspruch 6 sowie Anspruch 7, wobei die vorbestimmte Bedingung des Differenzflusses auf einer Integration des Differenzflusses über ein vorbestimmtes Integrationsintervall beruht.

9. Vorrichtung nach einem der vorangehenden Ansprüche wobei der Blutkreislauf, ein Teil des Blutkreislaufs, der Dialysierflüssigkeitskreislauf oder ein Teil des Dialysierflüssigkeitskreislaufs als Einmalartikel ausgeführt sind.

## Claims

1. A device (1, 2) for controlling or regulating an ultrafiltration in a dialysis treatment, in which the blood to be ultrafiltered flows through a blood chamber (110) of a dialyzer (113) that is subdivided by a semipermeable membrane (111) into the blood chamber (110) and a dialysis fluid chamber (108) in an extracorporeal blood circulation (112), and dialysis fluid in a dialysis fluid circulation (109) flows through the dialysis fluid chamber (108) of the dialyzer (113), having a blood pump (115) for generating a blood flow in the extracorporeal blood circulation (112), having a dialysis fluid pump (107) for generating a dialysis fluid flow in the dialysis fluid circulation (109), having a balancing device (104) for setting up a liquid balance in the dialysis fluid circulation between the inflow (106) and an outflow (105) of the dialysis fluid chamber (113) as a measure of the ultrafiltration, **characterized by**
a regulating unit (101) for regulating the blood pump (115) and/or the dialysis fluid pump (107), so that a predetermined ultrafiltration is achieved without further active control or regulation of the dialysis fluid flow coming out of the dialyzer, wherein the dialysis fluid pump (107) is arranged in an inlet line to the dialysis fluid chamber (113), and there is no further active control or regulation of the dialysis fluid flow downstream from the dialysis fluid chamber (113).

2.  The device (1, 2) for controlling or regulating an ultrafiltration according to claim 1, wherein the regulating unit (101) is adapted to predetermine an ultrafiltration rate and/or an ultrafiltration volume to be withdrawn during a course of treatment.

3.  The device (1, 2) according to any one of the preceding claims, wherein the blood pump is arranged in an inlet line to the blood chamber.

4.  The device (1, 2) according to any one of the preceding claims, wherein a predetermined value can be set for the blood flow and the regulating unit is adapted for regulating the dialysis fluid flow.

5.  The device (1, 2) according to any one of the preceding claims, wherein a predetermined value can be adjusted for the dialysis fluid flow, and the regulating unit is adapted for regulating the blood flow.

6.  The device (1, 2) according to any one of the preceding claims, wherein a profile for the ultrafiltration rate at which intervals of a positive ultrafiltration rate alternating with intervals of a negative ultrafiltration rate can be preselected by the regulating unit (101).

7.  The device (2) according to any one of the preceding claims, wherein the balancing device has a differential flow measuring unit (104) for measuring the differential flow between a flow in the inlet stream to the dialysis fluid chamber (108) and the outlet flow out of the dialysis fluid chamber, a branch from the inlet flow or the outlet flow to the branch of dialysis fluid from the inlet flow or the outlet flow into another flow path (212) as well as a device for adjusting the flow rate (211) in the incoming flow, the outgoing flow and/or the additional flow path, which can be controlled in such a way that the measured differential flow fulfills a predetermined condition and having a device (211) for determining the flow rate in the additional flow path as a measure of the fluid balance.

8.  The device (2) according to claim 6 as well as claim 7, wherein the predetermined condition of the differential flow is based on integration of the differential flow over a predetermined integration interval.

9.  The device according to any one of the preceding claims, wherein the blood circulation, a portion of the blood circulation, the dialysis fluid circulation or a portion of the dialysis fluid circulation is embodied as a disposable item.

**Revendications**

1.  Dispositif (1, 2) de commande ou de réglage d'une ultrafiltration dans un traitement par dialyse,
    dans lequel du sang à ultrafiltrer traverse dans un circuit sanguin extracorporel (112) une chambre à sang (110) d'un dialyseur (113) divisé par une membrane semi-perméable (111) en une chambre à sang (110) et une chambre à liquide de dialyse (108) et du liquide de dialyse traverse dans un circuit de liquide de dialyse (109) la chambre à liquide de dialyse (108) du dialyseur (113),
    comportant une pompe à sang (115) pour créer un flux sanguin dans le circuit sanguin extracorporel (112), une pompe à liquide de dialyse (107) pour créer un flux de liquide de dialyse dans le circuit de liquide de dialyse (109), comportant un dispositif pour déterminer un bilan entré - sortie de liquide dans le circuit de liquide dialyse entre une arrivée (106) et une évacuation (105) de la chambre à liquide dialyse (113) en tant qu'indice d'ultrafiltration,
    **caractérisé par** :

    une unité de réglage (101) pour régler la pompe à sang (115) et/ou la pompe à liquide de dialyse (107) de manière à obtenir une ultrafiltration prédéfinie sans autre commande active ou réglage du flux de liquide de dialyse s'écoulant du dialyseur, la pompe à liquide de dialyse (107) étant disposée dans une conduite d'alimentation de la chambre à liquide dialyse (113) et aucune autre commande active ou aucun réglage du flux de liquide de dialyse n'ayant lieu en aval de la chambre à liquide de dialyse (113).

2.  Dispositif (1, 2) de commande ou de réglage d'une ultrafiltration selon la revendication 1, dans lequel l'unité de réglage (101) est adaptée pour prédéfinir une vitesse d'ultrafiltration et/ou un volume d'ultrafiltration à extraire pendant un cycle de traitement.

**3.** Dispositif (1, 2) selon une des revendications précédentes, dans lequel la pompe à sang est disposée dans une conduite d'alimentation de la chambre à sang.

**4.** Dispositif (1, 2) selon une des revendications précédentes, dans lequel une valeur prédéfinie est réglable pour le flux sanguin et l'unité de réglage est adaptée pour régler le flux de liquide de dialyse.

**5.** Dispositif (1, 2) selon une des revendications précédentes, dans lequel une valeur prédéfinie est réglable pour le flux de liquide de dialyse et l'unité de réglage est adaptée pour régler le flux sanguin.

**6.** Dispositif (1, 2) selon une des revendications précédentes, dans lequel un profil de vitesse d'ultrafiltration est prescriptible par l'unité de réglage (101), dans lequel profil des intervalles à vitesse d'ultrafiltration positive et des intervalles à vitesse d'ultrafiltration négative alternent.

**7.** Dispositif (2) selon une des revendications précédentes, dans lequel le dispositif pour déterminer un bilan entré - sortie présente une unité de mesure de flux différentiel (104) pour mesurer le flux différentiel entre un flux dans l'arrivée à la chambre à liquide de dialyse (108) et l'évacuation de la chambre à liquide 2dialyse, une bifurcation de l'arrivée ou de l'évacuation pour dévier du liquide de dialyse depuis l'arrivée ou l'évacuation dans un autre parcours d'écoulement (212), ainsi qu'un dispositif de réglage de la quantité d'écoulement (211) dans l'arrivée, dans l'évacuation et/ou dans l'autre parcours d'écoulement et qui peut être commandé de manière à ce que le flux différentiel mesuré remplisse une condition prédéfinie et comportant un dispositif (211) de détermination de la quantité d'écoulement dans l'autre parcours d'écoulement sous forme d'indice du bilan entré - sortie du liquide.

**8.** Dispositif (2) selon la revendication 6 et la revendication 7, dans lequel la condition prédéfinie du flux différentiel repose sur une intégration du flux différentiel par un intervalle d'intégration prédéfini.

**9.** Dispositif selon une des revendications précédentes dans lequel le circuit sanguin, une partie du circuit sanguin, le circuit de liquide de dialyse ou une partie du circuit de dialyse sont réalisés sous forme d'articles à usage unique.

Fig. 1

EP 2 956 188 B1

Fig. 2

EP 2 956 188 B1

Fig. 3

EP 2 956 188 B1

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0006172 A1
- DE 3600227 A1
- EP 0516152 A1